Europäisches Patentamt

European Patent Office

Office européen des brevets

(19)

(11) EP 0 995 012 B1

(12) EUROPÄISCHE PATENTSCHRIFT

(45) Veröffentlichungstag und Bekanntmachung des Hinweises auf die Patenterteilung:
**21.03.2001 Patentblatt 2001/12**

(21) Anmeldenummer: **98939618.9**

(22) Anmeldetag: **06.07.1998**

(51) Int Cl.7: **E21B 37/06**

(86) Internationale Anmeldenummer:
**PCT/EP98/04158**

(87) Internationale Veröffentlichungsnummer:
**WO 99/04138 (28.01.1999 Gazette 1999/04)**

(54) **ETHERCARBONSÄUREN ALS ASPHALTEN-DISPERGATOREN IN ROHÖLEN**

ETHER CARBOXYLIC ACIDS AS ASPHALT DISPERSANTS IN CRUDE OILS

ACIDES CARBOXYLIQUES ETHERIQUES UTILISES COMME DISPERSANTS DANS DES HUILES BRUTES

(84) Benannte Vertragsstaaten:
**DE FR GB IT SE**

(30) Priorität: **14.07.1997 DE 19730085**

(43) Veröffentlichungstag der Anmeldung:
**26.04.2000 Patentblatt 2000/17**

(73) Patentinhaber: **Clariant GmbH**
**65929 Frankfurt am Main (DE)**

(72) Erfinder:
• **MILLER, Dennis**
**D-65779 Kelkheim (DE)**
• **VOLLMER, Axel**
**D-65830 Kriftel (DE)**
• **FEUSTEL, Michael**
**D-55278 Köngernheim (DE)**
• **KLUG, Peter**
**D-63762 Grossostheim (DE)**

(56) Entgegenhaltungen:
**EP-A- 0 207 312**      **US-A- 5 021 498**

EP 0 995 012 B1

**Beschreibung**

Asphaltene sind Bestandteile von Rohölen. Sie enthalten eine Vielzahl von Strukturen, besonders hochmolekulare kondensierte aromatische Komponenten mit Heteroatomen. Angesichts der Komplexität ihrer Chemie werden Asphaltene als die Ölfraktion beschreiben, die in Benzol, aber nicht in n-Pentan löslich ist.

Im Rohöl liegen Asphaltene normalerweise als kolloidale Dispersion vor. Diese wird durch Ölharze stabilisiert.

Asphaltene können während der Produktion, der Raffination, des Transports und der Lagerung von Rohöl und davon abgeleiteten Produkten, wie z.B. schweres Heizöl oder Schiffsöl, ausfallen. Gemeinsame Ursachen für dieses Ausfallen sind ein Absinken der Temperatur oder ein Wechsel in der Zusammensetzung (z.B. Verdampfung von leicht flüchtigen Bestandteilen). Asphaltene können auch beim Fließen durch poröse Medien ausfallen. Fluten mit $CO_2$ während des Förderprozesses kann Asphaltene zum Flokkulieren oder zum Ausfallen bringen.

Manche Öle enthalten Kohlenwasserstoffwachse, die bei niedrigen Temperaturen ausfallen. Wechselwirkungen zwischen dem Ausfallen von Wachs und Asphaltenen können die Gesamtmenge an ausgefallener Substanz oder deren Bildungsgeschwindigkeit erhöhen.

Ausgefallene Asphaltene verursachen Probleme bei der Produktion und bei der Verarbeitung von Rohölen. Asphaltene schlagen sich nieder in Ventilen, Rohren und Fördereinrichtungen. An heißen Oberflächen, wie beispielsweise Wärmetauschern, kann die Carbonisierung dieser Niederschläge ihre Entfernung sehr schwierig machen. Die Niederschläge reduzieren den Wirkungsgrad von Anlagen und können im schlimmsten Fall zu einer kompletten Blokkierung und zu einem Produktionsstop führen, was hohe Kosten verursacht.

Schweröle, die oft zum Antrieb von Schiffen verwendet werden, enthalten beträchtliche Mengen an Asphaltenen. Das Ausfallen von Asphaltenen kann sowohl zu schlechter Verbrennung als auch zu Schwierigkeiten bei der Handhabung und bei der Lagerung des Treibstoffes führen.

Bitumen, Schweröle und Rückstände werden manchmal mit Lösemitte verdünnt, um die Viskosität für den Transport zu reduzieren. Wenn dabei Asphaltene ausfallen, so ergeben sich damit Probleme bei der Handhabung.

Das Ausfallen von Asphaltenen kann durch kleine Mengen an Dispergatoren verhindert oder verringert werden. Diese Substanzen zeigen einen oder mehrere der folgenden Effekte:

a) Die Menge an Niederschlag wird reduziert;
b) der Niederschlag bildet sich langsamer;
c) der Niederschlag ist feiner verteilt; und
d) die Neigung des Niederschlages, sich auf Oberflächen abzulagern, wird reduziert.

Wenn sich bereits Niederschläge an Asphaltenen gebildet haben, können sie durch den Gebrauch von Lösemitteln entfernt werden. Die Zugabe eines Dispergators kann die Wirksamkeit dieser Lösemittel verbessern.

Eine Vielzahl von Asphalten-Dispergatoren sind bereits bekannt. CA 2 029 465 und CA 2 075 749 beschreiben Alkylphenolformaldehydharze in Kombination mit hydrophilen-lipophilen Vinylpolymeren. Die Asphalten-dispergierenden Eigenschaften von Dodecylbenzolsulfonsäure wurden beschrieben in US 4 414 035, außerdem durch D.-L. Chang und H.S. Fogler (SPE paper No. 25185) und durch M.N. Bouts et al. (J. pet. Technol. 47, 782-7, 1995).

EP-A-0 207 312 offenbart ein Verfahren zur Gewinnung von Öl aus unterirdischem Speichergestein, indem eine Lösung oder Dispersion eines carboxymethylierten Oxalkylates in Mischung mit einem hydrophoberen Tensid eingepreßt wird. Die Verwendung von carboxymethylierten Oxalkylaten allein oder in Mischung mit anderen Bestandteilen als Asphaltendispergator wird nicht offenbart.

Die bisher bekannten Dispergatoren können die durch das Ausfallen von Asphaltenen verursachten Probleme nur teilweise lösen. Da Öle in ihrer

Zusammensetzung variieren, können einzelne Dispergatoren nur in einem beschränkten Bereich wirksam arbeiten. Manchmal haben sogar kleine Änderungen in der Ölzusammensetzung einen großen Effekt auf die Dispergiereigenschaften für Asphaltene. Deshalb sind in einigen Fällen die bekannten Dispergatoren nicht zufriedenstellend und zusätzliche Typen sind erforderlich.

Es bestand somit die Aufgabe, neue Asphalten-Dispergatoren zur Verfügung zu stellen, die die beschriebenen Nachteile der bisher bekannten Dispergatoren nicht aufweisen.

Überraschenderweise wurde gefunden, daß Ethercarbonsäuren der Formel

$$RO(CH_2CHR_1O)_x(CH_2CHR_2O)_yCH_2CO_2H$$

verwendet werden können, um das Ausfallen und/oder das Ablagern von Asphaltenen in Rohölen und davon abgeleiteten Produkten zu verhindern.

Gegenstand der Erfindung sind somit Rohöle und davon abgeleitete Produkte enthaltend als Asphaltendispergator Ethercarbonsäuren der Formel

$$RO(CH_2CHR_1O)_x(CH_2CHR_2O)_yCH_2CO_2H$$

worin

R $C_6$-$C_{22}$-, vorzugsweise $C_9$-$C_{18}$-Alkyl oder -Alkenyl, $C_6$-$C_{20}$-Alkylaryl,
$R_1$ und $R_2$ unabhängig voneinander H oder Methyl, vorzugsweise H bedeuten, und
x und y unabhängig voneinander eine Zahl von 0 bis 20 sind, wobei die Summe aus
x und y 1 bis 20, vorzugsweise 1,5 bis 8 beträgt.

Von Rohölen abgeleitete Produkte sind beispielsweise schweres Heizöl, Schiffsöl oder Bitumen.

Die erfindungsgemäßen Ethercarbonsäuren können neben anderen Methoden durch Umsetzung von Alkylphenol oder von Fettalkoholen natürlichen oder synthetischen Ursprungs mit Ethylenoxid und/oder Propylenoxid zu den entsprechenden oxalkylierten Alkoholen und anschließender Reaktion mit Alkali (Natriumhydroxid, Kaliumhydroxid) und Natriumchloracetat bzw.

Chloressigsäurederivaten hergestellt werden; zur Isolierung der Ethercarbonsäuren wird anschließend angesäuert und die Ethercarbonsäure von der salzhaltigen Wasserphase getrennt.

Die so erhaltenen Produkte sind Mischungen von Molekülen mit verschiedenen Längen der Polyalkylenoxidketten. Die Zahlen x und y sind deshalb als Mittelwerte zu verstehen.

Geeignet für die Dispergierung von Asphaltenen sind sowohl reine Ethercarbonsäuren als auch deren technische Qualitäten, die neben Ethercarbonsäuren (in Anteilen > 50 %, bevorzugt 60-90 %) meist noch den zugrundeliegenden oxalkylierten Alkohol (in Anteilen von 1-40 %, bevorzugt 5-30 %) und Wasser (in Anteilen < 20 %, bevorzugt 2-10 %) enthalten. Bei den in den Beispielen genannten Ethercarbonsäuren handelt es sich um solche technische Qualitäten, die zwischen 60 und 80 % reine Ethercarbonsäure enthalten.

Der erfindungsgemäße Dispergator wird in einer Konzentration von 0,5 bis 10.000 ppm, vorzugsweise von 2 bis 2.000 ppm eingesetzt.

Zur leichteren Dosierung kann der Dispergator als Lösung in einem ölmischbaren Lösemittel formuliert werden, wie beispielsweise aromatische Kohlenwasserstoffe oder Mischungen von Kohlenwasserstoffen und einem aliphatischen Alkohol.

Zusätzlich zu dem erfindungsgemäßen Dispergator können auch Alkylphenolformaldehydharze, oxalkierte Amine, Wachsdispergatoren oder beliebige Mischungen daraus verwendet werden. Ebenfalls können andere organische Säuren mit Tensideigenschaften wie z.B. Mono- oder Dialkylbenzolsulfonsäuren, Petrolsulfonsäuren und Alkansulfonsäuren als zusätzliche Komponenten eingesetzt werden.

Prüfung der Wirksamkeit von Asphaltendispergatoren

Prinzip des Dispergiertestes

Die Dispergierung, das Ausfällen von Asphaltenen hängt von der Natur des Kohlenwasserstoffmediums ab. Asphaltene sind in aromatischen, aber nicht in aliphatischen Kohlenwasserstoffen löslich. Somit können Dispergatoren getestet werden, indem man das Öl oder extrahierte Asphaltene in einem aromatischen Lösemittel löst und indem man dann einen aliphatischen Kohlenwasserstoff zugibt, um einen Niederschlag zu erzeugen. Da Asphaltene von dunkler Farbe sind, kann das Ausmaß des Niederschlages durch eine kolorimetrische Messung der überstehenden Flüssigkeit bestimmt werden. Je dunkler die überstehende Flüssigkeit ist, desto mehr Asphaltene bleiben dispergiert, d.h. umso besser ist der Dispergator. Dieser Test wird beschrieben in CA 2 029 465. In unserer Version des Tests wird das Fällungsmedium so ausgewählt, daß die Asphaltene zum größten Teil, aber nicht komplett ausfallen.

Vorschrift Dispergiertest

a) Eine 25 %ige Öl-Lösung in Toluol wird filtriert, um Verunreinigungen zu beseitigen;
b) 9,5 ml Heptan als Fällungsmittel für Asphaltene und 0,5 ml Toluol/Dispergator-Mischung (25:1) in ein gut 10 ml fassendes graduiertes Glasröhrchen vorlegen und gut schütteln. Dies entspricht einer Dispergatorkonzentration von 2000 ppm. Bei Bedarf kann die Menge Dispergator variiert werden. Für die Nullproben wird reines Toluol verwendet;
c) in das Glasröhrchen wird dann 0,1 ml von der gefilterten Öl-Lösung dazugegeben und ebenfalls gut geschüttelt;
d) das Ganze 2 Stunden ohne Erschütterungen stehenlassen. Die ausgefällten Asphaltene sollen sich am Boden des Röhrchens sammeln können;

e) nach Ablauf dieser Zeit wird das Volumen des Sediments an Hand der Graduierung abgeschätzt, das Aussehen der gesamten Probe protokolliert und dann wird von der überständigen Phase 1 ml vorsichtig mit einer Pipette aufgenommen;

f) die abgesaugte Menge wird in 5 ml einer 99:1 Toluol-Triethanolamin-Mischung gelöst und bei 600 nm photometriert.

Bewertung des Dispergiertests

Als relatives Maß für die Dispergierung wird folgender Ausdruck genommen

$$A = 100 \ (D-D_0)/D_0,$$

wobei D und $D_0$ optische Dichte von Meßlösung und Blindprobe sind. Der maximal erreichbare Wert von A, $A_{max}$, entspricht vollständiger Dispergierung der Asphaltene. Sie kann abgeschätzt werden, indem ein Versuch ohne Dispergator, mit Toluol anstatt Heptan, durchgeführt wird - dadurch bleiben die Asphaltene vollständig dispergiert. Das Volumen des Sediments liefert eine weitere Information über die Wirksamkeit des Dispergators. Je kleiner die Menge an Sediment ist, desto besser dispergiert ist die Substanz.

Beispiele

Mit einem asphaltenreichen Öl aus Venezuela wurden erfindungsgemäße Substanzen mit dem Dispergiertest geprüft. Die Dosis betrug 2000 ppm.

| Nr. | R | $R_1$ | x | y | Dispergierwirkung A [%] | Sedimentvolumen ml |
|---|---|---|---|---|---|---|
| 1 | Oleyl | H | 2 | 0 | 108 | 0 |
| 2 | $C_{14/5}$ Alkyl | H | 3 | 0 | 110 | 0 |
| 3 | $C_{14/5}$ Alkyl | H | 7 | 0 | 106 | 0 |
| 4 | $C_{12}$ Alkyl | H | 3 | 0 | 112 | 0 |
| 5 | i-$C_9$Arylalkyl | H | 4 | 0 | 115 | 0 |
| Null Probe | -- | -- | -- | -- | 0 | 0,45 |

Bei dieser Versuchsreihe betrug die maximale Dispergierwirkung $A_{max}$ ca. 120 %

**Patentansprüche**

1. Rohöle und davon abgeleitete Produkte, enthaltend als Asphalten-Dispergator Ethercarbonsäuren der allgemeinen Formel

$$RO(CH_2CHR_1O)_x(CH_2CHR_2O)yCH_2\text{-}CO_2H$$

worin

R $C_6$-$C_{22}$-Alkyl oder -Alkenyl, $C_6$-$C_{20}$-Alkylaryl,
$R_1$ und $R_2$ unabhängig voneinander H oder Methyl bedeuten, und
x und y unabhängig voneinander eine Zahl von 0 bis 20 sind, wobei die Summe aus x und y 1 bis 20 beträgt.

2. Rohöle gemäß Anspruch 1, dadurch gekennzeichnet, daß

R $C_9$-$C_{18}$-Alkyl oder -Alkenyl, $C_6$-$C_{20}$-Alkylaryl,
$R_1$ und $R_2$ gleichzeitig H und
x und y unabhängig voneinander eine Zahl von 0 bis 20 sind, wobei die Summe aus x und y 1,5 bis 8 beträgt.

3. Verwendung von Ethercarbonsäuren gemäß Anspruch 1 als Asphalten-Dispergatoren in Rohölen und davon abgeleiteten Produkten.

4. Verfahren zum Dispergieren von Asphaltenen in Rohölen und davon abgeleiteten Produkten, dadurch gekennzeichnet, daß Ethercarbonsäuren gemäß Anspruch 1 in einer Menge von 0,5 bis 10.000, vorzugsweise von 2 bis 2.000 ppm zugegeben werden.

5. Verfahren gemäß Anspruch 4, dadurch gekennzeichnet, daß zusätzlich Alkylphenolformaldehydharze, oxalkierte Amine, Mono- oder Dialkylsulfonsäuren, Petrolsulfonsäuren, Alkansulfonsäuren, Wachsdispergator oder beliebige Mischungen daraus verwendet werden.

**Claims**

1. A crude oil and product derived therefrom comprising, as asphaltene dispersant, an ethercarboxylic acid of the formula

$$RO(CH_2CHR_1O)_x(CH_2CHR_2O)yCH_2\text{-}CO_2H$$

where

R is $C_6$-$C_{22}$-alkyl or -alkenyl, $C_6$-$C_{20}$-alkylaryl,
$R_1$ and $R_2$ independently of one another are H or methyl, and
x and y independently of one another are a number from 0 to 20, the sum of x and y being 1 to 20.

2. A crude oil as claimed in claim 1, wherein

R is $C_9$-$C_{18}$-alkyl or -alkenyl, $C_6$-$C_{20}$-alkylaryl,
$R_1$ and $R_2$ are simultaneously H and
x and y independently of one another are a number from 0 to 20, the sum of x and y being 1.5 to 8.

3. The use of ethercarboxylic acids as claimed in claim 1 as asphaltene dispersants in crude oils and products derived therefrom.

4. A process for dispersing asphaltenes in crude oils and products derived therefrom, which comprises adding ethercarboxylic acids as claimed in claim 1 in an amount of 0.5 to 10,000, preferably 2 to 2000, ppm.

5. The process as claimed in claim 4, wherein, in addition, use is made of alkylphenol-formaldehyde resins, alkoxylated amines, mono- or dialkylsulfonic acids, petroleumsulfonic acids, alkanesulfonic acids, wax dispersants or any mixtures thereof.

**Revendications**

1. Huiles brutes et produits dérivés, contenant comme agent dispersant des asphaltènes des acides alcoxycarboxyliques de formule générale

$$RO\,(CH_2CHR_1O)_x(CH_2CHR_2O)_yCH_2CO_2\text{-}H$$

dans laquelle

R  représente un groupe alkyle ou alcényle en $C_6$ à $C_{22}$, (alkyle en $C_6$ à $C_{20}$)aryle
$R_1$ et $R_2$  représentent indépendamment l'un de l'autre un atome d'hydrogène ou un groupe méthyle, et
x et y  représentent indépendamment l'un de l'autre un nombre compris entre 0 et 20, la somme de x et de y étant entre 1 et 20.

**2.** Huiles brutes selon la revendication 1, caractérisées en ce que

R représente un groupe alkyle ou alcényle en $C_9$ à $C_{18}$, (alkyle en $C_6$ à $C_{20}$)aryle,

$R_1$ et $R_2$ représentent en même temps un atome d'hydrogène, et

x et y représentent indépendamment l'un de l'autre un nombre compris entre 0 et 20, la somme de x et de y étant entre 1,5 et 8.

**3.** Utilisation d'acides alcoxycarboxyliques selon la revendication 1 comme agents dispersants d'asphaltènes dans des huiles brutes et de produits dérivés.

**4.** Procédé de dispersion d'asphaltènes dans des huiles brutes et produits dérivés, caractérisé en ce qu'on ajoute des acides alcoxycarboxyliques selon la revendication 1 en une quantité variant de 0,5 à 10000, de préférence de 2 à 2000 ppm.

**5.** Procédé selon la revendication 4, caractérisé en ce que, l'on utilise en plus des résines alkylphénolformaldéhyde, des amines alxoxylées, des acides mono- ou dialkylsulfoniques, des acides pétroléosulfoniques, alcanesulfoniques, des agents dispersants de cire ou des mélanges quelconques de ceux-ci.